# EUROPEAN PATENT SPECIFICATION

(11) **EP 3 200 758 B1**
(45) Date of publication and mention of the grant of the patent: **10.04.2019**
(21) Application number: 15784189.1
(22) Date of filing: 30.09.2015
(51) Int. Cl.: A61K 8/39, A61K 8/02, A61Q 9/02, A61Q 19/00, A61Q 5/00

(54) **ERODIBLE ANHYDROUS POLYETHYLENE OXIDE FILM**
ERODIERBARER ANHYDRISCHER POLYETHYLENOXIDFILM
FILM D'OXYDE DE POLYÉTHYLÈNE ANHYDRE ÉRODABLE

(30) Priority: 30.09.2014 US 201462057487 P
(43) Date of publication of application: 09.08.2017
(73) Proprietor: Edgewell Personal Care Brands, LLC, Chesterfield, MO 63017 (US)
(72) Inventor: SMITH, James A, Chatham, MA 02633 (US); KELLETT, George, Cranford, NJ 07016 (US); MEHTA, Rooma, Guilford, CT 06437 (US); MCCORMACK, Robert, Orange, CT 06477 (US); BURGIO, Paul, St. Paul, MN 55110 (US)
(74) Representative: dompatent von Kreisler Selting Werner - Partnerschaft von Patent- und Rechtsanwälten mbB
(86) International application number: PCT/US2015/053154
(87) International publication number: WO 2016/054167

(56) References cited:
- EP-A2- 2 206 490
- WO-A1-97/02116

## Description

### TECHNICAL FIELD

This invention relates generally to an erodible anhydrous polyethylene oxide film, and uses for such films. The erodible anhydrous films are useful in delivering active agents in a consistent and sustainable manner to substrates over multiple uses.

### BACKGROUND OF THE INVENTION

The background of this invention most closely relates to the field of shaving, but this invention has application to other uses where delivery of active agents to a substrate is desired, such as delivering therapeutic agents to keratinous surfaces, e.g., surfaces of skin or hair, or cleaning agents to a substrate.

Delivering therapeutic agents to skin are known. For instance, shaving devices are capable of simultaneously removing hair and applying a shaving aid material to the skin that provides lubriciousness during shaving and moisturization post shaving. Specifically, some razors utilize a cartridge head that includes a shaving aid strip mounted on a skin-contacting surface of the cartridge. This shaving aid strip, sometimes referred to as a "comfort strip," releases shaving aid materials when wet including skin care agents, as the razor cartridge is stroked over a user's skin.

Such devices having these strips are popular and have achieved significant commercial success. Even small improvements that benefit comfort, performance, usable life, ease of use, or overall skin care, can boost the commercial success of the device. For example, because the shaving aid strip is consumed long before the razor blades, this limits the usable life of a razor cartridge unnecessarily.

Existing strips include a water-insoluble polymeric matrix having a lubricious material and/or other skin care agents physically entrapped within the polymeric matrix. Polyethylene oxide (PEO) is a known lubricious material, and has been used in shaving aid comfort strips. As the molecular weight of the PEO increases, its lubricity increases. However, the PEO from conventional shaving aid strip has disadvantages. Amongst the most significant is that PEO swells dramatically when exposed to water. If the PEO swells too much prior to or during shaving, this creates an undesirable change in the cartridge geometry that controls the position of the blade as it cuts the hair. The result is a loss of closeness and a lower performing shaving cartridge. Also, traditional methods of making the PEO comfort strip involves high heat that degrades the PEO wherein the end product contains PEO with a significantly lower molecular weight than the starting PEO.

Another problem is the sustained and prolonged release rate of the PEO. In the context of shaving, these shaving aid strips produced either by conventional methods typically last no more than 5 shaves. Furthermore, extrusion or injection molding requires skin care agents that can withstand high temperature processing.

WO 97/02116 discloses a shaving aid comprising PEO having a portion of PEO with MW of 5 million, a water-insoluble polymer such as high impact polystyrene, PEG, active agent, colorant and antioxidant. The strips are made by melt-extrusion. Other water-insoluble polymers are polyethylene, polypropylene, polystyrene, polystyrene-butadiene copolymer, polyacetal, acrylonitrile-butadienestyrene copolymer, and ethylene vinyl acetate copolymer and the water-soluble polymer is selected from the group consisting of polyethylene oxide, polyvinyl pyrrolidone, polyacrylamide, hydroxypropyl cellulose, polyvinyl imidazoline, and polyhydroxyethylmetharcrylate. Therefore, there is a need to deliver a sustained and consistent release of PEO and other active agents to a substrate surface, and a method of manufacturing the comfort strip that reduces the degradation of the PEO and other skin care agents.

### SUMMARY OF THE INVENTION

The present invention provides erodible anhydrous films capable of sustained and consistent release of PEO and other active agents to a substrate surface, compositions and methods useful for preparing such films, and articles and devices comprising the films. In the present erodible anhydrous films, the PEO serves multiple primary and ancillary functions, serving as active agent, e.g., lubricant and emollient, an erodible carrier facilitating transfer of other active agents, a dispersant, a film forming agent, etc.

In particular, the present invention is directed to the following embodiments:
1. An erodible anhydrous film comprising, based on a total weight of said erodible anhydrous film,
   40 wt. % to 80 wt. % of polyethylene oxide, wherein at least a portion of the polyethylene oxide is one or more polyethylene oxide polymers having a molecular weight of 2,000,000 to 10,000,000;
   5 wt. % to 40 wt. % of one or more support polymers; and
   one or more agents selected from therapeutic agents, cleaning agents, or processing aids,
   characterized in that the one or more support polymers comprises one or more (meth) acrylate/(meth)acrylic acid copolymer, monoalkyl ester of polymethyl vinyl ether/maleic anhydride copolymer, vinyl pyrrolidone/ dimethylaminoethylmethacrylate copolymer, behenyl methacrylate/*t*-butyl methacrylate copolymer, vinyl acetate/butyl maleate/ isobornyl acrylate terpolymer, alone or in combination thereof.
2. The erodible anhydrous film of embodiment 1, wherein the therapeutic agents comprise one or more of aloe, vitamins, humectants, emollients, moisturizers, clotting agents, anti-chafing agents, fragrances, depilatory agents, essential oils, antioxidants, alpha-hydroxy acids, alpha-keto acids, anti-bacterials, anti-fungals, anti-microbials, anti-virals, analgesics, anti-allergenics, antihistamines, anti-inflammatory agents, anti-irritants, anti-neoplastics, immune boosting agents, immune suppressing agents, anti-acne agents, anti-aging agents, anesthetics, antiseptics, insect repellents, skin cooling compounds, skin warming compounds, skin protectants, skin penetration enhancers, exfoliants, lubricants, or combinations thereof.
3. The erodible anhydrous film of embodiment 1 or 2, wherein the cleaning agents comprise de-greasers, surfactants, enzymes, fabric conditioners, anti-microbials, anti-bacterials, anti-fungals, or combinations thereof, and
   the processing aids comprise plasticizers, which plasticizers comprise polyethylene glycol, propylene glycol, butylene glycol, pentalene glycol, glycerin or combinations thereof, or wherein the processing aids comprise flexibilizers, which flexibilizers comprise silicone resins, waxes, elastomers, polyamides, or combinations thereof, or
   wherein the processing aids comprise fillers, which fillers comprise boron nitride, graphite, talc, clay, silicates, diatomaceous earth, or combinations thereof.
4. The erodible anhydrous film of embodiment, comprising a combination of PEO with polyvinylpyrrolidone and/or glycerol and/or polyalkylene glycol.
5. The erodible anhydrous film of embodiment 1, wherein the one or more support polymer is present in an amount of 6 wt. % to 35 wt. %.
6. The erodible anhydrous film of any one of embodiments 1 through 5 obtained by a process comprising extruding, injection molding, and or compression molding an anhydrous composition comprising said polyethylene oxide, said one or more support polymers, and said one or more active agents selected from therapeutic agents, cleaning agents and /or processing aids.
7. A process for making the erodible anhydrous film of any one of embodiments 1 through 6 said process comprising
   providing an anhydrous fluid composition comprising,
   polyethylene oxide, wherein at least a portion of the polyethylene oxide is one or more polyethylene oxide polymers having a molecular weight of 2,000,000 to 10,000,000 daltons;
   one or more support polymer, wherein the one or more support polymers comprises one or more (meth)acrylate/(meth)acrylic acid copolymer, monoalkyl ester of polymethyl vinyl ether/maleic anhydride copolymer, vinyl pyrrolidone/ dimethylaminoethylmethacrylate copolymer, behenyl methacrylate/*t*-butyl methacrylate copolymer, vinyl acetate/butyl maleate/isobornyl acrylate terpolymer, alone or in combination thereof;
   one or more active agents selected from therapeutic agents, cleaning agents, or processing aids; and
   one or more anhydrous solvent, wherein the one or more anhydrous solvent is present in an amount of 15 wt. % to 55 wt. %, based on a total weight of the anhydrous fluid composition;
   casting the erodible anhydrous film by pouring the anhydrous fluid composition onto a substrate or into a mold or into a cavity in an end use device or a component of an end use device; and
   drying the anhydrous fluid thus cast.
8. The process of embodiment 7, wherein the one or more solvent of the anhydrous fluid composition comprises one or more C₂ to C₁₂ alcohols, one or more C₅ to C₂₀ alkanes, one or more C₅ to C₂₀ alkenes, or one or more ethers.
9. The process of embodiment 7 further comprising water in an amount of less than 5 wt. %, based on a total weight of said anhydrous fluid composition.
10. A multi-layer film comprising one or more layers of the erodible anhydrous film of any one of embodiments 1 through 6.
11. A skin care wipe, a wound care device, hair care device, fabric care device or an erodible shaving aid material comprising the erodible anhydrous film of any one of embodiuments 1 through 6.
12. A hair removal device comprising the erodible shaving aid material of embodiment 11.

Also disclosed but not claimed is:
13. a method of delivering active agents to a surface comprising the steps of
   providing a device comprising an erodible anhydrous film of any one of embodiments 1 through 6; and
   activating the device by exposure to water or water vapor; and
   contacting the surface with the activated device such that the polyethylene oxide and the one or more active agents are released onto the surface.
14. The method of embodiment 13, wherein the step of providing the device comprises providing one of a hair care device, a hair removal device, a wound care device, a fabric care device or a skin care wipe.
15. The method of embodiment 13, wherein the step of contacting the surface comprises contacting a keratinous surface, a hard surface or a fabric.

Also disclosed is an anhydrous composition for forming an erodible anhydrous film comprising polyethylene oxide and one or more support polymers. One embodiment, for example, provides an anhydrous fluid composition comprising (i) polyethylene oxide present in an amount of 2.5 wt. % to 50 wt. %; (ii) one or more support polymers; and (iii) one or more anhydrous solvents, wherein the anhydrous fluid composition forms an erodible anhydrous film when the solvent is removed. In some other embodiments, an anhydrous composition for forming an erodible anhydrous film is provided which contains little to no added solvent, i.e., an anhydrous non-solvent composition, comprising (i) polyethylene oxide in an amount of 40 wt. % to 80 wt. %, for example, 40 or 50 wt. % to 70 or 80 wt. %, based on a total weight of the anhydrous composition, and (ii) one or more support polymers.

Typically, the one or more support polymers comprise copolymers selected from the group consisting of (meth)acrylate/(meth)acrylic acid copolymers, monoalkyl esters of polymethyl vinyl ether/maleic anhydride copolymer, vinyl pyrrolidone/dimethylaminoethylmethacrylate copolymer, behenyl methacrylate/*t*-butylmethacrylate copolymer, vinyl acetate/butyl maleate/isobornyl acrylate terpolymer, alone or in combination. (Meth)acrylate means acrylate or methacrylate. The one or more support polymer is typically present in the anhydrous fluid composition in an amount of 5 wt. % to 40 wt. %. In the anhydrous composition containing little to no added solvent, the one or more support polymer is typically present in an amount of 6 wt. % to 45 wt. %.

The one or more solvents in the anhydrous fluid composition may be present in an amount of 15 wt. % to 55 wt. %. Preferably, the one or more solvents comprises one or more C₂ to C₁₂ alcohols, i.e., C₂ to C₁₂ linear alcohols or isomers thereof, ethers, such as tetrahydrofuran, or one or more C₅ to C₂₀ alkanes or alkenes, i.e., C₅ to C₂₀ linear alkanes or alkenes or isomers thereof. In some embodiments, the one or more solvent comprises ethanol, isopropanol, or isododecane.

The anhydrous composition for forming an erodible anhydrous film may further comprise active agents, cleaning agents, processing aids, or combinations thereof. Preferably, the active agents are therapeutic active agents and comprise one or more of aloe, vitamins, humectants, emollients, moisturizers, clotting agents, anti-chafing agents, fragrances, depilatory agents, essential oils, antioxidants, alpha-hydroxy acids, alpha-keto acids, anti-bacterials, anti-fungals, anti-microbials, anti-virals, analgesics, anti-allergenics, antihistamines, anti-inflammatory agents, anti-irritants, anti-neoplastics, immune boosting agents, immune suppressing agents, anti-acne agents, anti-aging agents, anesthetics, antiseptics, insect repellents, skin cooling compounds, skin warming compounds, skin protectants, skin penetration enhancers, exfoliants, lubricants, or combinations thereof. Preferably, the cleaning agents comprise de-greasers, surfactants, enzymes, fabric conditioners, anti-microbials, anti-bacterials, anti-fungals, or combinations thereof. The processing aids may comprise of emulsifiers, plasticizers, solubilizers, flexibilizers, rheology modifiers, fillers, or combinations thereof. In various embodiments the processing aids are plasticizers comprising polyethylene glycol, propylene glycol, butylene glycol, pentalene glycol, glycerin or combinations thereof; flexibilizers comprising silicone resins, waxes, elastomers, polyamides, or combinations thereof; and/or fillers comprising boron nitride, graphite, talc, clay, silicates, diatomaceous earth, or combinations thereof. In some embodiments, the anhydrous composition further comprises water in an amount of about less than 5 wt. %, based on a total weight of the composition.

The anhydrous fluid composition may comprise polyethylene oxide in an amount of 4.5 wt. % to about 45 wt. %, based on a total weight of the anhydrous fluid composition; the one or more support polymers mentioned above; and
one or more solvents comprising C₂ to C₁₂ alcohols.

In some embodiments of the anhydrous fluid composition, the one or more support polymers comprise cycloalkyl methacrylate copolymer and the solvent comprises isododecane.

In another aspect, the present invention is directed to an erodible anhydrous film comprising (i) polyethylene oxide in an amount of 40 wt. % to 80 wt. % based on a total weight of the erodible anhydrous film; for example, 40, or 50 wt. % to 70 or 80 wt. % and (ii) from 5 wt. to 45 wt. % of one or more support polymers mentioned above. comprising acrylate or methacrylate polymers, acrylate copolymers, methacrylate.

For example, the one or more support polymers comprise (meth)acrylate polymers or copolymers, vinyl acetate copolymers, polyvinyl alcohol copolymers, vinyl ether copolymers, polyurethanes, polyvinylpyrrolidone, vinylpyrrolidone copolymers, maleic anhydride copolymers, high impact styrene polymers, polypropylene, acrylonitrile butadiene styrene (ABS) polymer, or combinations thereof.

As mentioned above the one or more support polymers comprise copolymers such as (meth)acrylate/(meth)acrylic acid copolymers, monoalkyl esters of polymethyl vinyl ether/maleic anhydride copolymer, vinyl pyrrolidone/dimethylaminoethylmethacrylate copolymer, behenyl methacrylate/*t*-butyl methacrylate copolymer, vinyl acetate/butyl maleate/isobornyl acrylate terpolymer, alone or in combination thereof, for example, (meth)acrylate/(meth)acrylic acid copolymers or vinyl acetate/butyl maleate/isobornyl acrylate copolymer.

Typically, the polyethylene oxide is present in an amount of 40 or 50 wt. % to 70 or 80 wt. %, and the one or more support polymers are present in an amount of 5, 6, or 9 wt. % to 25, 29, 35, or 40 wt. %, e.g., 6 to 29, 35, or 40 wt. %, based on a total weight of the erodible anhydrous film.

The erodible anhydrous film may further comprise active agents, cleaning agents, processing aids, and combinations thereof. Preferably, the active agents are therapeutic active agents and comprise one or more of aloe, vitamins, humectants, emollients, moisturizers, clotting agents, anti-chafing agents, fragrances, depilatory agents, essential oils, antioxidants, alpha-hydroxy acids, alpha-keto acids, anti-bacterials, anti-fungals, anti-microbials, anti-virals, analgesics, anti-allergenics, antihistamines, anti-inflammatory agents, anti-irritants, anti-neoplastics, immune boosting agents, immune suppressing agents, anti-acne agents, anti-aging agents, anesthetics, antiseptics, insect repellents, skin cooling compounds, skin warming compounds, skin protectants, skin penetration enhancers, exfoliants, lubricants, or combinations thereof. Preferably, the cleaning agents comprise de-greasers, surfactants, enzymes, fabric conditioners, anti-microbials, anti-bacterials, anti-fungals, or combinations thereof. The processing aids may comprise of emulsifiers, plasticizers, solubilizers, flexibilizers, rheology modifiers, fillers, or combinations thereof. Preferably, the plasticizers comprise polyethylene glycol, propylene glycol, butylene glycol, pentalene glycol, glycerin or combinations thereof. Preferably, the flexibilizers comprise silicone resins, waxes, elastomers, polyamides, or combinations thereof. Preferably, the fillers comprise boron nitride, graphite, talc, clay, silicates, diatomaceous earth, or combinations thereof.

In some embodiments, the erodible anhydrous film comprises one or more layers of the erodible anhydrous film to create a multi-layered film. In some embodiments, the erodible anhydrous film further includes, or is adhered to, a substrate.

In yet another aspect, the present invention is directed to erodible anhydrous films that may be part of a skin care wipe, a wound care device, a hair care device, a hair removal device, and a fabric care device.

In yet another aspect, the present invention is directed to an erodible shaving aid material comprising an erodible anhydrous film of the present invention. Preferably, the therapeutic agent comprises one or more of aloe, vitamins, humectants, emollients, moisturizers, clotting agents, anti-chafing agents, fragrances, depilatory agents, essential oils, antioxidants, alpha-hydroxy acids, alpha-keto acids, anti-bacterials, anti-fungals, anti-microbials, anti-virals, analgesics, anti-allergenics, antihistamines, anti-inflammatory agents, anti-irritants, anti-neoplastics, immune boosting agents, immune suppressing agents, anti-acne agents, anti-aging agents, anesthetics, antiseptics, insect repellents, skin cooling compounds, skin warming compounds, skin protectants, skin penetration enhancers, exfoliants, lubricants, or combinations thereof. The erodible anhydrous film of the shaving aid material may further include processing aids comprising plasticizers, solubilizers, flexibilizers, rheology modifiers, fillers, or combinations thereof.

In still yet another aspect, the present invention is directed to a hair removal device comprising the foregoing erodible shaving aid material.

In still yet another aspect, the present invention is directed to a method of delivering active agents to a surface comprising the steps of providing a device comprising an erodible anhydrous film as described above, comprising: (i) polyethylene oxide (ii) one or more support polymers; and (iii) one or more active agents comprising therapeutic agents or cleaning agents; activating the device by exposure to water or water vapor; and contacting the surface with the activated device such that the polyethylene oxide and the one or more active agents are released onto the surface.

Preferably, the step of providing the device comprises providing one of a hair care device, a hair removal device, a wound care device, a fabric care device or a skin care wipe.

Preferably, in the step of providing the device comprising an erodible anhydrous film, the therapeutic agents comprise aloe, vitamins, emollients, moisturizers, clotting agents, anti-chafing agents, fragrances, depilatory agents, essential oils, antioxidants, alpha-hydroxy acids, alpha-keto acids, anti-bacterials, anti-fungals, anti-microbials, anti-virals, analgesics, anti-allergenics, antihistamines, anti-inflammatory agents, anti-irritants, anti-neoplastics, immune boosting agents, immune suppressing agents, anti-acne agents, anti-aging agents, anesthetics, antiseptics, insect repellents, skin cooling compounds, skin warming compounds, skin protectants, skin penetration enhancers, exfoliants, lubricants, or combinations thereof. Preferably, in the step of providing the device comprising an erodible anhydrous film, the cleaning agents comprise de-greasers, surfactants, enzymes, fabric conditioners, anti-microbials, anti-bacterials, anti-fungals, or combinations thereof. Preferably, the step of providing the device comprising an erodible anhydrous film, the erodible anhydrous film further includes processing aids comprising emulsifiers, plasticizers, solubilizers, flexibilizers, rheology modifiers, fillers, or combinations thereof.

Preferably, the step of contacting the surface comprises contacting a keratinous surface, a hard surface, or a fabric.

The erodible anhydrous film of the invention can be prepared from the anhydrous composition comprising polyethylene oxide and one or more support polymers using well known processing techniques such as film casting, injection molding, compression molding, spray-coating, calendaring extrusion, co-extrusion, etc., depending on the formulation of the anhydrous composition and end use. For example, the erodible anhydrous film may be prepared by casting from an anhydrous fluid composition comprising a solvent as described above or by extrusion, compression molding or injection molding of a non-solvent anhydrous composition comprising little or no solvent.

### DETAILED DESCRIPTION OF THE PREFERRED EMBODIMENTS

The embodiments of the present invention can comprise, consist of, and consist essentially of the features and/or steps of the invention described herein, as well as any of the additional or optional ingredients, components, steps, or limitations described herein or would otherwise be appreciated by one of skill in the art. It is to be understood that all concentrations disclosed herein are by weight percent (wt. %) based on a total weight of the specified composition unless otherwise indicated. Where appropriate, the INCI (International Nomenclature of Cosmetic Ingredients) name of ingredients/components is used.

The present invention addresses the need for a consistent and sustained release of PEO and active agents through an erodible anhydrous film. The erodible anhydrous films and related embodiments described herein are quite effective in controlling the release rate of PEO and such active agents including, for example only, providing a substantially improved method of delivering PEO and/or other skin care agents to skin in a variety of applications. In certain embodiments, such as in the use of the erodible anhydrous film in a comfort strip of a razor, the release of PEO, along with other therapeutic agents contained therein, can be correlated with the lubrication provided by PEO transfer to the treated surface.

The "anhydrous composition" and "anhydrous fluid composition" of the invention contain no more than 5 wt. % water, typically less than 5 wt. % water, e.g., 2 wt. % or less water. The "erodible anhydrous film" of the invention is prepared from the "anhydrous composition" or "anhydrous fluid composition" and therefore also contains no more than 5 wt. % or less of water, typically 2 wt. % or less water, as prepared. Often, when the erodible anhydrous film is prepared from an anhydrous composition of the invention containing a small amount of water, for example, when prepared by film casting from an anhydrous fluid composition comprising up to 5 wt. % water, the erodible anhydrous film produced will comprise less water than the precursor composition because some if not all of the water will be removed as the film is dried or otherwise processed.

It is of course understood that in many end uses the erodible anhydrous film will be exposed to, and absorb, water as part of its designed function, for example, as a comfort strip of a razor that is swelled by exposure to water immediately prior to contacting the users skin. However, the erodible anhydrous film itself, and the article comprising the film, e.g. the comfort strip, will result in a product comprising no more than 5 wt. % water.

The anhydrous film provided by the invention is "erodible" meaning that under conditions of standard use, the film wears or loses mass due to mechanical abrasion caused by contacting the film with a surface. The film erodes as a whole such that all the essential components of the film, e.g., PEO, support polymer, other active agents, cleaning agents, processing aids and the like, that contact the surface, are transferred together to the surface. The film remaining after contact maintains the same relative composition of components as existed prior to contact. For example, the amount of support polymer in the remaining film does not increase relative to the amount of PEO during use, because both PEO and support polymer are directly transferred in combination to the contacting surface.

This is not to say that each individual component of the film in isolation would be considered erodible. For example, certain active components of the inventive film can be readily dissolved in water and lost from the film, however, such components under standard use conditions remain entrained in the PEO/support polymer matrix of the present erodible anhydrous film. Conversely, some of the support polymers useful in the present invention, e.g., polyolefins such as polypropylene, styrenics such as high impact polystyrene, other polymers and blends such as ABS, would not typically be considered to erode upon contact with skin or hair, however, when processed according to the invention into the present erodible anhydrous films, these polymer are dispersed throughout the bulk PEO composition in a manner in which they can offer structural support to the film, but are transferred along with the rest of the film matrix to the surface being contacted.

It is should be noted that extensive exposure to water or other solvents can dissolve the erodible anhydrous film, or dissolve and leach components of the film, however, as can be readily gleaned from the present specification, this would not generally be considered "standard use". For example, in many embodiments of the invention, the erodible anhydrous film is exposed to water during or immediately prior to contacting a surface. For example, a razor, and the comfort strip thereon, is typically wetted before contacting the skin; a sponge or cleansing cloth will also be wetted before contacting skin, hair or a hard surface such as a countertop, dish, car hood, etc. A high molecular weight PEO will swell when exposed to water, such as during shaving, and will impart a lubricious film when contacted to a surface. However, abusive treatment of the film, e.g., soaking the film in water over an extended period of time will eventually lead to loss of the PEO due to dissolution. However, this would not be considered standard conditions of use.

Conditions of standard use for an erodible anhydrous film of the invention in many embodiments may therefore comprise exposing the film to water for a period of time sufficient to wet the film or to allow for absorption of water by the film, prior to contacting a surface, but in most embodiments the film would not remain in contact with water for a period of time that would allow for complete or near complete dissolution and leaching of film components into the water. As stated above, the amount of time of water exposure needed to wet the film for a particular end use is well within the knowledge of even the casual user of many of the common end use products for which the inventive erodible anhydrous films are intended. If deemed necessary, instructions for use of the erodible anhydrous film or article comprising the film can be provided. Non-standard use of the erodible anhydrous films are envisioned, but standard end use conditions under which the film is used are those wherein the film is contacted to a surface after exposing the film to water for a brief time to wet the film or to activate the film via swelling due to water absorption followed by contacting the wet film to a surface.

In the presence of a small amount of water and adequate pressure, the erodible anhydrous film when contacted against the skin during, for example, wet shaving, can deliver consistent and sufficient levels of PEO and other active agents while maintaining its dimensional and structural integrity. The erodible anhydrous film of the present invention transfers mass from the surface of the film as it contacts the targeted substrate so that it should be understood that "dimensional integrity" as used herein refers to the dimensions of the erodible anhydrous film other than the diminution of thickness due to wear at the contacting surface. Utilizing the technology disclosed herein, the erodible anhydrous films may be manufactured so as to control the number of uses by a consumer to deliver satisfactory amounts of the PEO or other active agents (e.g., shaving aid or hair removal aid materials). For instance, the erodible anhydrous films when used as an erodible comfort strip are contemplated to be effective for about 1 to about 24 or even 40 uses. Almost any number of uses within this range can be engineered, for example, 2 to about 40 uses, 2 to 12, 2 to 5, 4 to 12, 5 to 12 or 5 to 10 uses. Modifying the thickness and composition of the erodible anhydrous film makes it possible to "dial in" the number of uses that can be delivered by this technology. For instance, the erodible anhydrous films are contemplated to have a thickness of about 0.1 mm to about 75 mm although the thickness of the erodible anhydrous film will depend upon its use in a final product. For example, in some embodiments the erodible anhydrous film of the invention will have a thickness of from about 0.1 mm, 0.25 mm or 0.5 mm to about 10 mm or 25 mm, e.g., about 0.25 to about 5 mm, e.g., 1 mm, 1.5 mm, 2 mm, 3 mm, 3.5 mm, 4 mm 4.5 mm etc.

As disclosed herein, the size, thickness and chemical composition can be adjusted to serve the purpose desired, in various combinations. Very advantageously, therefore, the versatility of the erodible anhydrous films accommodates, for example, a sustained and consistent amount of PEO released during a shave as well as the ability to control the number of shaves the comfort strip can deliver.

In one embodiment the erodible anhydrous film starts as a fluid composition that may comprise or consist essentially of PEO, one or more support polymers, and a solvent for dispersing the PEO and the support polymers. Once the PEO and support polymers are dispersed in the solvent, they are mixed until homogenous and processed whereupon the solvent is removed to form a substantially uniform dried film comprising or consisting of the PEO and the support polymer. Additional components such as active agents and processing aids may be added during the mixing phase to alter rheology or improve resultant film qualities. The dispersion is then cast and the solvent is removed, leaving an erodible anhydrous film. The erodible anhydrous film is then generally cut into an appropriate size and shape suitable for the intended end use.

It is also possible to use the solvent casting of a film as a means to generate a formulated composition comprising PEO, a support polymer and other optional components as an intermediate which is then subjected to further processing or shaping. For example, the solid cast film may be ground to produce granules or a powder, which is then formed into a final article by extrusion, injection molding compression molding or other common processing method, during which additional process components may optionally be added to the PEO, erodible anhydrous film containing composition.

In another embodiment the erodible anhydrous film is prepared from a solid formulation or predominately solid formulation comprising or consisting essentially of PEO, one or more support polymers, and other additional optional components of the invention, which solid or predominately solid formulation is then subjected to additional processing, e.g., extrusion, injection molding or compression molding, to form the final erodible anhydrous film. A variety of mixing or blending techniques are known in the art for preparing the starting solid or predominately solid formulation. Molding techniques useful in the present invention may involve melt processing techniques wherein the composition is in a liquid molten state during processing, or in some processes, for example, certain compression molding processes, are intimately mixed, but the solid or predominately solid composition is formed into the inventive erodible anhydrous film without dissolution or melting.

### Polyethylene oxide

As mentioned above, the PEO of the erodible anhydrous film comprises one or more polyethylene oxides with a number average molecular weight of 2,000,000 daltons to 8,000,000 daltons or even to 10,000,000 daltons, to help ensure the lubricity of a resultant product such as a shaving aid comfort strip. More than one polyethylene oxide material may be used and all of the PEO or a portion of the PEO present in the film may comprise the high molecular weight material, while in some embodiments a portion of the PEO may be a material with a lower molecular weight, less than 2,000,000 daltons.

The combination of PEO and the support polymer is critical to the function and effectiveness of the films formed from an anhydrous fluid composition of the invention. In the dried erodible anhydrous film, this combination facilitates the erosion of the support polymer at a desirable rate, and allows the PEO to be released at a consistent and sustained rate. Furthermore, PEO acts as the main viscosity modifier in the fluid composition to make a substantially continuous erodible anhydrous film. Without the presence of PEO in the fluid composition, an acceptable film cannot be as readily formed. When PEO is present in amounts above 50 wt. %., the fluid composition is undesirably viscous, and again it is difficult to form a substantially continuous film.

To that end, the fluid composition should have an ideal viscosity level wherein the fluid composition can flow to form a substantially continuous film of substantially uniform thickness. Ideally, viscosity of the fluid composition is between
the fluid composition is conveniently measure by a Brookfield LV viscometer, e.g., in the present application the viscosities were determined using a Brookfield LV viscometer at 25°C with a #2 spindle at 2 rpm. After drying the film, outer edges of the film may be trimmed to remove areas where the thickness may be unacceptably less than a central portion of the film.

In the fluid composition, the PEO is preferably present in an amount of 2.5 wt. % to 50 wt. %, based on a total weight of the fluid composition. In preferred embodiments of the fluid composition, the PEO is present between 4 or 4.5 wt. % to 45 wt. %, and more preferably from 30 wt. % to 40 wt. %. After removing the solvent from the fluid composition to form the erodible anhydrous film, the PEO is present in an amount of 40 wt. % to 80 wt. %, and more preferably from 40 or 50 wt. % to 70 or 80 wt. %, based on a total weight of the dried erodible anhydrous film.

Depending on how the erodible anhydrous film is used in a final product will dictate the particle size of the PEO used in the fluid composition. One of ordinary skill in the art can determine the requisite particle size for a particular application without undue experimentation as PEO is available from different suppliers in different particle sizes. PEO is readily available at a particle size between 50 µm and 700 µm.

Similarly, the combination of PEO and erodible support polymer plays a significant role in the function and effectiveness of the erodible anhydrous films formed from a composition containing little to no added solvent. While viscosity of a casting solution is not a factor in these embodiments, it is advisable to choose formulation components that have, for example, acceptable melting points and handling characteristics, and produce mixtures that transform smoothly under the processing conditions being used.

Furthermore, the final product may also dictate the molecular weight of the PEO in forming the erodible anhydrous film. As an example only, PEO having a molecular weight between 2 million and 10 million is particularly useful as a lubricious shaving aid. PEO having mixtures of different molecular weights may also be useful in treating other keratinous surfaces.

PEO may be commercially available as POLYOX® from The Dow Chemical Company, Midland, Michigan, in a variety of molecular weights and particle sizes. As known in the industry and references in POLYOX® commercial literature, the molecular weight of POLYOX® polyethylene oxides such as those used herein, are determined largely by viscosity data.

Other components that can function in ways similar to PEO, for example, polyvinylpyrrolidone and various glycerol and polyalkylene glycol materials known to have lubricity characteristics similar to PEO, may also be present, however, the erodible anhydrous films of the invention are more readily formed and perform in a more controlled and effective manner due to the presence of the PEO. For example, in some embodiments combinations of PEO, polyvinylpyrrolidone and/or glycerol and/or polyalkylene glycol are present.

### Support Polymer

The support polymers are another essential element of the invention. The support polymer should be compatible with PEO, be capable of forming high quality film when combined with the PEO, and should erode along with the PEO during use. When used in an anhydrous fluid composition the support polymer should be dissolvable/dispersible in a non-aqueous solvent so as to produce an acceptable erodible anhydrous film. It functions to assist in creating a support for PEO and other components in the erodible anhydrous film. The support polymer helps to form a solid but erodible film, allows PEO and other components to be released from the erodible anhydrous film when in contact with adequate amounts of water, controls the swelling of the PEO, and maintains dimensional and structural integrity of the resultant erodible anhydrous film. Preferably, the support polymer is able to support the controlled delivery of the PEO and other active agents to a desired surface. Preferably, this polymer has high temperature stability, provides reasonable viscosity in the fluid composition for efficient film casting, and provides a resultant erodible anhydrous film that is smooth and non-tacky.

In preferred embodiments of the fluid composition, the support polymer is present in an amount of 5 wt. % to 40 wt. %, and more preferably between 7.5 wt. % to 35 wt. %, and most preferably from 10 wt. % to 30 wt. %, all based on a total weight of the fluid composition. After the solvent is removed from the fluid composition, the support polymer is present in the erodible anhydrous film in an amount of 6 wt. % to 40 wt. %, often from 6 wt. % to 29 wt. %, and more preferably from 9 wt. % to 25 wt. %, all based on a total weight of the erodible anhydrous film. In anhydrous compositions containing little to no added solvent, the support polymer is present in amounts similar to those found in the erodible anhydrous film.

According to the invention, the one or more support polymers are selected from (meth)acrylate/(meth)acrylic acid copolymers, monoalkyl esters of polymethyl vinyl ether/maJeic anhydride copolymer, vinyl pyrrolidone/dimethylaminoethylmethacrylate copolymer, behenyl methacrylate/*t*-butyl methacrylate copolymer, vinyl acetate/butyl maleate/isobornyl acrylate terpolymer, alone or in combination thereof, for example, (meth)acrylate/(meth)acrylic acid copolymers or vinyl acetate/butyl maleate/isobornyl acrylate copolymer.

Typically, the support polymers are present in the erodible anhydrous film in an amount of 5, 6 or 9 wt. % to 25, 29, 35 or 40 wt. %, e.g., 6 to 29 or 6 to 35 wt. %, based on a total weight of the erodible anhydrous film.

Vinyl pyrrolidone copolymers useful in the present invention are commercially available, for example, under, e.g., the trade name GAFQUAT® from Ashland Inc., Covington, Kentucky. For example, GAFQUAT® 734, is a vinyl pyrrolidone/ dimethylaminoethylmethacrylate copolymer quaternized with diethylsulfate in ethanol.

Vinyl ether/maleic anhydride copolymers useful in the present invention are commercially available, for example, under, e.g., the trade name GANTREZ®, from Ashland Inc., as monoalkyl esters of polymethyl vinyl ether/maleic anhydride copolymers. These include the ethyl, butyl, isopropyl esters of methyl vinyl ether/maleic anhydride copolymers in an alcoholic solution. Another such copolymer is the monoethyl ester of polymethyl vinyl ether/maleic anhydride available under the trade name OMNIREZ® from Ashland Inc. Acrylate and methacrylate polymers and copolymers useful in the present invention include alkyl and cycloalkyl acrylate and methacrylate polymers, e.g., polymers made from C₁₋₂₄ alkyl or C₅₋₁₂ cycloalkyl acrylate or methacrylate monomers, which monomers are optionally substituted with hydroxyl, alkoxy or amino groups; acrylate and methacrylate co-polymers made from mixtures of such acrylate and/or methacrylate monomers, co-polymers made from made from mixtures of acrylate and/or methacrylate monomers with acrylic and/or methacrylic acid monomers; co-polymers with non-acrylate or methacrylate monomers, such as vinyl pyrrolidone/dimethylaminoethylmethacrylate copolymers, vinyl acetate/butyl maleate/isobornyl acrylate terpolymers, and the like, alone or in combination. These acrylate support polymers are commercially available under, e.g., the trade names EUDRAGIT® available from EVONIK; GIOVAREZ® from Phoenix Chemical Inc., Somerville, New Jersey; ADVANTAGE® PLUS, also from Ashland Inc., alone or in combination therewith. For example, acrylate and methacrylate polymers and copolymers useful in the anhydrous fluid composition include isododecane solution of cycloalkyl methacrylate sold as GIOVAREZ® 5099M or isododecane (and) behenyl methacrylate/*t*-butylmethacrylate copolymer sold as GIOVAREZ® BTB-50. The ADVANTAGE® PLUS is vinyl acetate/butyl maleate/isobornyl acrylate terpolymer in ethanol.

Polyurethanes useful in cosmetic and personal care compositions are known and can be used in the present invention, e.g., polyurethanes based on polyether, polyester and/or polycaprolactone polyols. Certain particular embodiments employ a polyurethane polymer soluble in solvents such as tetrahydrofuran or other organic solvents. In one embodiment, an anhydrous fluid composition comprising THF, a soluble polyurethane and PEO is prepared and cast to form an erodible anhydrous film of the invention.

Thermoplastic polymers such as polypropylene, high impact polystyrene, ABS and the like can also be used as the support polymer. Such polymers are particularly useful in forming erodible anhydrous films wherein particles or small segments of the thermoplastic are dispersed within a largely PEO substrate. This type of film is conveniently made by compression molding of a well-blended composition, comprising the polymer, PEO and other optional active components, for example a blended mixture of solids or a mixture comprising a molten PEO and solid polymer. Other processing techniques may also be used including extrusion, injection molding, etc.

### Solvent

The solvent is an essential ingredient of the fluid compositions and assist in forming the erodible anhydrous films disclosed herein. The solvent should be non-aqueous, and preferably solubilizes the support polymer. It should not dissolve the PEO, but allows the PEO to be well dispersed in the fluid composition. Additionally, it should be volatile at room temperature, e.g., 25° Celsius (C), to 200 °C. Preferably, the solvent will be able to dissolve one or more of the processing aids described herein although a dispersion of the processing aids is also acceptable. The solvent is preferably either an alcohol, ether or a hydrocarbon, depending on the choice of support polymer selected. Other solvents can be used as long as they meet the above criteria. Several of the support polymers disclosed herein are commercially available in the preferred solvents.

The choice of solvent may depend on its flash point. Preferably the flash point is low enough to meet OSHA requirements for safety, but high enough to handle a higher drying temperature of the film if that is desired. One of ordinary skill in this art would know how to optimize the solvent for production purposes.

Alcohols chosen as the solvent are preferably C₂ to C₁₂ alcohols or isomers thereof. Examples include ethanol, propanol, butanol, pentanol, hexanol, heptanol, octanol, nonanol, decanol, undecanol, dodecanol, and their isomers. Preferred alcohols are ethanol, isopropanol, alone or in combination. Ethers, such as THF, can also be used in preparing the fluid compositions. Hydrocarbon solvents are preferably C₅ to C₂₀ alkanes, C₅ to C₂₀ alkenes, or isomers thereof, for example in one embodiment the hydrocarbon solvent is isododecane.

Preferably, the solvent is present in the fluid composition in an amount or quantity sufficient to provide the appropriate and desired consistency to form a substantially continuous film. For example, the solvent is present in an amount of 15 wt. % to 55 wt. %, e.g., of 20 wt. % to 50 wt. %,.

### Optional Components - Active Agents and Processing Aids

As desired, the fluid composition, non-solvent composition, i.e., composition of the invention containing little to no solvent, and resultant erodible anhydrous film can include other components as described herein. An important criteria is that the additional components themselves do not contain water (other than trace amounts), so as to preserve the anhydrous nature of the compositions and resultant erodible anhydrous film.

Besides PEO, active agents that enhance performance and comfort of the erodible anhydrous film can be incorporated therein. These active agents should be either at least partially soluble, miscible with, or dispersible in the solvent or compatible with the processing conditions employed when forming an erodible anhydrous film from a non-solvent composition, should not hinder the formation of the erodible anhydrous film, and should be released from the erodible anhydrous film in the presence of adequate amounts water so as to be deliverable to the desired surface.

Examples of active agents that are therapeutic to keratinous surfaces include aloe, vitamins (e.g., A, C or E), humectants, emollients, moisturizers, clotting agents, anti-chafing agents, fragrances, depilatory agents, essential oils, antioxidants, alpha-hydroxy acids, alpha-keto acids, anti-bacterials, anti-fungals, anti-microbials, anti-virals, analgesics, anti-allergenics, antihistamines, anti-inflammatory agents, anti-irritants, anti-neoplastics, immune boosting agents, immune suppressing agents, anti-acne agents, anti-aging agents, anesthetics, antiseptics, insect repellents, skin cooling compounds, skin warming compounds, skin protectants, skin penetration enhancers, exfoliants, lubricants, and the like.

When the erodible anhydrous film is incorporated into a hair removal device, the compositions and erodible anhydrous films of the invention may include such shaving aids such as lubricating agents for reducing the drag forces between the razor and the skin; depilatory agents that may modify the chemical structure of the hair to allow the razor blade to pass through the whiskers very easily; surfactants or cleaning agents that allow the whiskers and skin debris to be washed more easily from the razor parts during shaving; medicinal agents; cosmetic agents for softening, smoothing, conditioning, cooling, heating, or improving the tone or texture of skin; blood clotting agents to suppress bleeding that may occur from nicks and cuts; and astringents for constricting blood vessels thereby stemming the flow of bodily fluids such as lymph, which may exude from skin which has been irritated during shaving.

For erodible anhydrous films outside of personal care products, active agents include, but are not limited to, cleaning agents for hard and soft surfaces, de-greasers, surfactants, enzymes, fabric conditioners, water conditioners, anti-microbials, anti-bacterials, anti-fungals, and the like. Surfactants can be anionic, cationic, non-ionic, or zwitterionic.

Processing aids that assist or improve the processability, solubility, flexibility, viscosity, or performance of the erodible anhydrous film itself can be added during formulation or processing of the fluid or non-solvent compositions. These may or may not be water soluble, but do form a part of the erodible anhydrous film. Examples of these processing aids are emulsifiers, plasticizers, solubilizers, flexibilizers, rheology modifiers, fillers, and the like. These additives are present in the erodible anhydrous film in an amount of 0.5 wt. % to 15 wt. %, for example 1 to 10 wt.% or 1 to 5 wt. %.

The following specific examples of processing aids are non-limiting, as would be understood by someone having ordinary skill in this art. These are included to further explain and illustrate the wide breadth of ingredients that are useful in the erodible anhydrous films disclosed herein. It is possible that some ingredients can perform more than one function, as would be understood by one of ordinary skill in this art.

Examples of useful plasticizers include polyethylene glycol having a molecular weight between 200 to 20,000, propylene glycol, butylene glycol, pentalene glycol, or glycerin. Examples of useful flexibilizers include silicone resins, waxes, elastomers, polyamides, and the like. A preferred silicone resin is SILFORM® resin from Momentive Performance Materials Inc., Waterford, New York. A preferred group of polyamide polymers are commercially available under the trade name SYLVACLEAR® from Arizona Chemical, Jacksonville, Tennessee. Examples of useful elastomers include silicone elastomer gels such as isodecyl neopentanoate (and) dimethicone/*bis*-isobutyl PPG-20 crosspolymer; cyclopentasiloxane (and) dimethicone crosspolymer (and) dimethicone/vinyl dimethicone crosspolymer (and) dimethiconol. Both are commercially available from Dow Corning Corporation, Midland, Michigan.

A non-limiting example of a dual purpose additive may be an emollient such as isostearyl neopentanoate that also acts as a plasticizer. Such emollients are commercially available from Ashland Inc. under the trade name CERAPHYL®.

Examples of fillers useful in the erodible anhydrous film include boron nitride, graphite, talc, clay, silicates, diatomaceous earth, or combinations thereof.

Optionally, water in an amount of less than about 5 wt. %, preferably less than about 1 wt. %, based on a total weight of the fluid composition, can be added to the fluid composition as a viscosity modifier. Upon drying the film, the water substantially evaporates leaving negligible, if any, amounts of water left in the resultant erodible anhydrous film.

### Erodible Anhydrous Film Preparation

Erodible anhydrous films of the invention can be prepared by a variety of known film forming techniques including solvent casting, spraying, calendaring, extrusion, co-extrusion injection molding, blow molding, compression molding, and the like. The specific processing techniques will depend on the end use of the erodible anhydrous film and the particular components, including optional active components, being used. Particular embodiments of the invention include forming the erodible anhydrous films by solvent casting, extrusion, injection molding and compression molding, forming the films using a mixture of two or more of these processes, e.g., solvent casting followed by injection or compression molding of the ground cast film, and the films produced thereby.

In one particular embodiment, the erodible anhydrous films are produced by a process comprising solvent casting, e.g., casting the erodible anhydrous film from the anhydrous fluid composition.

The fluid compositions can conveniently be prepared by charging a vessel, typically equipped with a heating element and stirrer, with the solvent. While stirring, the components of the fluid compositions, e.g., support polymer, PEO, optional active components, etc., are added to the solvent until a homogeneous dispersion is achieved. Heating may be required to assure that particular components such as certain polymers, actives or processing aids are solubilized in the solvent, if desired. Low shear mixing is generally utilized when the mixture contains higher molecular weight PEO to preserve the molecular integrity of the polymer and prevent degradation.

For example, to a beaker containing isopropanol is added one or more plasticizers, e.g., SYLVACLEAR® and/or SILFORM® resin, with heating, e.g., to about 45 °C, until the plasticizer is dissolved. The beaker can be covered to minimize evaporation of the isopropanol. The solution can then be cooled to room temperature if desired, held at the current temperature of heated higher. A support polymer, e.g., an acrylate or methacrylate polymer or copolymer, is added with mixing. Depending on the support polymer, the polymer may dissolve or become suspended in the solvent. If the polymer is suspended, stirring should be continued until a evenly dispersed suspension is obtained. Powdered PEO is then slowly added until the PEO is fully dispersed in the mixture with little clumping. Optional processing aids and/or active components, such as, polyethylene glycol-200, CERAPHYL® 375 emollient, skin care agents such as aloe and Vitamin E, etc., can then be added, individually or separately, with stirring. Additional isopropanol is added in a quantity sufficient to replace the amount evaporated during heating and/or adjust the viscosity to 2000 MPa●s (cPs) to 5000 MPa●s (cPs).

The erodible anhydrous film can then be formed by pouring the fluid composition onto a substrate or into a mold and then evaporating the solvent, typically by heating. For example, the fluid composition can be poured onto a glass or metal plate, e.g., stainless steel or aluminum plate etc., which may optionally be fitted with a sheet of silicone treated release paper on the top side. The fluid can then be drawn down using an appropriate doctor blade to obtain a desired thickness, and the cast fluid composition can then be dried, e.g., in a convection oven at 80 °C to 85 °C until a constant weight is achieved. Obviously different temperatures may be used depending for example on the solvent boiling point. Using the appropriate doctor blade, one of ordinary skill in the art can readily produce dried erodible anhydrous films having thicknesses of 1 mm, 1.5 mm, 2 mm, etc., which can then be cooled and cut to the desired size.

The fluid composition may also be cast directly onto the end use device or a component that is used in preparing an end use device, or cast directly into a cavity of the device or device component, dried and then put into use as is.

One of skill in the art will have the knowledge to cast the fluid composition onto an appropriate substrate to achieve a film of desired thickness. Substrates contemplated herein include woven and non-woven substrates, foam substrates, adhesive backed substrates, polymer substrates, and casting onto a layer of the dried erodible anhydrous film itself to produce a multi-layered film. The solvent can be removed by heating the cast dispersion in an oven set for a time and temperature sufficient to volatize the solvent and solidify the film or by other means known to one of ordinary skill in the art.

In another particular embodiment the erodible anhydrous films are produced by a process comprising injection molding or compression molding of a blended mixture of components. Such processes are typically most successful when liquid components are excluded or used in low amounts and absorbed by the dry ingredients in the blended mixture. Any process for mixing or blending the components of the erodible anhydrous film can be used including simple dry blending, high speed mixing, milling etc. Solvent cast films from above can be milled or granulated to provide the blended mixture of components that are molded or extruded.

Injection molding and compression molding can be accomplished using a simple blend of the components, e.g., PEO, support polymer, optional actives and processing aids, etc., however, better results are often obtained if the components are first thermally processed, e.g., Brabender melt mixing or extrusion, typically followed by granulation or pelletization.

Extrusion, either as a prelude to an additional molding process or as a means of directly forming the erodible anhydrous film can be carried out using a single or double screw extruder with barrel temperatures of from 80 to 230 depending on the PEO and support polymer used. To injection mold the erodible anhydrous films, it is preferred to first extrude the formulated blend into pellets using e.g., a single screw extruder at a temperature of 90°C to 200°C or 220°C, e.g., 120°C to 180°C or 100 to 150°C, then injecting the pellets in either a single material molding or multi-material molding machine optionally equipped with a hotrunner system using temperatures of from 120°C to 250°C, e.g., from 140°C to 220°C, at an injection pressure sufficient to fill the part completely without flashing, which depending on the cavity size, configuration and quantity can range from 2.07 to 17.2 MPa (300 to 2500 psi). The cycle time is dependent on the same parameters and can range from 3 to 30 seconds.

For compression molding, the formulated anhydrous non-solvent composition can be prepared via extrusion or in a mixer at ambient or elevated temperature. For example, the compositions can be compounded in a Banbury or other mixer at elevated temperature then formed into a sheet, which sheet can be cut into strips or plaques that are subsequently compression molded. In one embodiment, an intimately mixed composition comprising the support polymer, PEO and optional active components can be compression molded directly, e.g., using a commercial compression molding apparatus such as a Carver press, to form a composite erodible anhydrous film of the invention.

In many embodiments, the fluid composition used to make the erodible anhydrous film comprises materials shown in Table I below:

**Table I**

| **Component** | **Amount (wt. %)** |
|---|---|
| PEO | 25 - 41 |
| Erodible Support Polymer | 4 - 30 |
| Solvent | 25 - 40 |
| Flexibilizer | 1 - 3 |
| Elastomer | 1 - 2 |
| Plasticizer | 4 - 6 |
| Skin Care Agents | 0.5 - 5 |

In many embodiments the non-solvent composition to make the erodible anhydrous film and the erodible anhydrous film itself comprises materials as shown in Table Ia below:

**Table Ia**

| **Component** | **Amount (wt. %)** |
|---|---|
| PEO | 40 - 80 |
| Support Polymer | 6 - 40 |
| Flexibilizer | 1 - 5 |
| Elastomer | 1 - 4 |
| Plasticizer | 5 - 10 |
| Skin Care Agents | 0.5 - 10 |

In some exemplary embodiments, the erodible anhydrous film has a thickness of from about 0.1 to about 25 mm or 0.1 to 10 mm, and is prepared by a process comprising solvent casting, extrusion, injection molding and/or compression molding.

For example, in one set of exemplary embodiments, the erodible anhydrous film is prepared by solvent casting from an anhydrous fluid composition comprising: polyethylene oxide, wherein at least a portion of the polyethylene oxide is one or more polyethylene oxide polymer having a molecular weight of 2,000,000 to 10,000,000; one or more support polymer, wherein the one or more support polymers comprises a methacrylate polymer, acrylate copolymer, methacrylate copolymer, vinyl acetate polymer or copolymer, polyvinyl alcohol polymer or copolymer, vinyl ether copolymer, polyvinylpyrrolidone, vinylpyrrolidone copolymer, or maleic anhydride copolymer, a blend comprising said polymers or copolymers, or a combination thereof; one or more active agent selected from therapeutic agents, cleaning agents and /or processing aids; one or more anhydrous solvent, wherein the one or more anhydrous solvent is present in an amount of 15 wt.% to 55 wt. %, based on the total weight of the anhydrous fluid composition, which anhydrous fluid composition may also comprise water, e.g., 0 to 5 wt. % water, typically less than 5 wt. % water.

In many of these exemplary solvent cast embodiments, the one or more solvents of the anhydrous fluid composition comprises one or more C₂ to C₁₂ alcohols, one or more C₅ to C₂₀ alkanes, one or more C₅ to C₂₀ alkenes, or one or more ethers, often one or more C₂ to C₁₂ alcohols, or one or more C₅ to C₂₀ alkanes. In the solvent cast embodiments, the one or more support polymers of the anhydrous fluid composition, and the resulting erodible anhydrous film, comprises one or more (meth)acrylate/(meth)acrylic acid copolymer, monoalkyl ester of polymethyl vinyl ether/maleic anhydride copolymer, vinyl pyrrolidone/dimethylaminoethylmethacrylate copolymer, behenyl methacrylate/*t*-butyl methacrylate copolymer, vinyl acetate/butyl maleate/isobornyl acrylate terpolymer, alone or in combination thereof.

In other of these exemplary embodiments, the erodible anhydrous film of the invention is obtained by a process-comprising extrusion, injection molding, and or compression molding an anhydrous composition comprising: the polyethylene oxide as described above, one or more support polymers as described above and one or more active agent selected from therapeutic agents, cleaning agents and /or processing aids.

### Devices and Methods of Use

The erodible anhydrous films disclosed herein are useful in a variety of applications, such as personal care, fabric care, and hard surface cleaning and treating, just to name a few. In one embodiment, the erodible anhydrous film can be integrated into or associated with a hair removal device, such as a razor, a razor cartridge, an epilator, alone or in combination with a light source to degrade or destroy the hair and/or hair root. If the hair removal device comprises a light source, then the light source may generate coherent (laser) light or incoherent light and may be adapted to generate light continuously or in a discontinuous (pulsed) fashion.

Preferably, the hair removal device is a razor. In the case of a razor, the erodible anhydrous film may be arranged, positioned or otherwise provided on the razor cartridge as a comfort strip. For instance, the comfort strip can be preferably located before and/or after the blade(s) in the direction of cutting and may even be configured as a continuous portion entirely surrounding the blades. WO 97/02116 illustrates locations in which such a strip may be placed.

The erodible anhydrous film may be affixed to the hair removal device in any appropriate fashion. For instance, it can be mechanically fitted onto a separate razor cartridge, or directly onto the hair removal device. Alternatively, the erodible anhydrous film may be directly or indirectly adhered to the hair removal device by means of an adhesive composition. One method of indirect adherence involves casting the solid film onto an adhesive backed sheet, for example, an acetate sheet, as a possible substrate, which is then adhered to the hair removal device via the adhesive.

In one exemplary embodiment, a comfort strip for a razor comprises an erodible anhydrous film of the invention, which film comprises
40-80 wt. % PEO;
6 to 40 wt. %, or 6 to 35 wt. % support polymer, such as an acrylate or methacrylate polymer or copolymer, e.g., a(meth)acrylate copolymer or (meth)acrylate/(meth)acrylic acid copolymer and the like;
1 to 25 wt. % or 1 to 20 wt.% of one or more plasticizers, flexiblizers or processing aids, such as glycerol based or fatty acid based processing aids, polyethylene glycol (PEG), polyamide, citrate, silicone or siloxane plasticizers/flexiblizers, and the like; and
up to 10 wt.% skin care ingredients such as aloe, vitamins, emollients, moisturizers, clotting agents, anti-chafing agents, fragrances, depilatory agents, essential oils, antioxidants, anti-irritants, antiseptics, skin cooling compounds, protectants, other lubricants and the like.

In shaving and hair removal devices, the erodible anhydrous film can controllably and consistently deliver PEO and active agents such as shaving aid materials and skin care agents to the skin or hair before, during or after shaving. Once the erodible anhydrous films described herein containing PEO and the active agents are exposed to an adequate amount of water, the PEO and active agents are released onto the skin or hair as the shaving or hair removal device bearing the erodible anhydrous film is moved across the skin. As the erodible anhydrous film moves across the skin, the film erodes away to provide a fresh surface on which the active agents are readily accessible. This is an improvement over prior art extruded or molded comfort strips wherein the PEO is trapped within a water-insoluble matrix.

Another embodiment of the erodible anhydrous film entails delivering wound care materials to the skin. The user may use one of the erodible anhydrous films described herein and, after or simultaneously being exposed to adequate amounts of water, place the erodible anhydrous film onto an open wound on the skin or on scratched or abraded skin. The PEO and wound care materials are released upon contact directly onto the skin. Wound care materials will promote clotting, healing, deliver therapeutic agents, or relieve discomfort associated with wounds, rashes, abrasions, cuts, etc.

When used in the context of skin treatment, the erodible anhydrous film can be fitted into a hand-held device for delivery of skin care agents to the skin surface. For instance, the erodible anhydrous films can be replaceable in the skin treatment device. For example, in a skin treatment for preventing or treating acne, it is preferred that the amount of PEO in the liquid composition is 10 wt. % to 20 wt. %, preferably 15 wt. %, based on a total weight of the fluid composition. It is also preferred that the fluid composition contain about up to 5 wt. % salicylic acid, preferably about 1 wt. %, based on a total weight of the fluid composition, and at least one surfactant. A user would activate the erodible anhydrous film with an adequate amount of water then contact the device to that portion of their skin requiring treatment.

Another embodiment utilizing the erodible anhydrous film is a skin care wipe. When the erodible anhydrous films are in the form of a skin care wipe, a user sweeps the wipe across either wet skin or activates the wipe with water to moisten the erodible anhydrous film just prior to use, so that water-soluble active agents within the wipe, including the PEO, are released onto the skin in the area desired. Preferably, one such embodiment is a moisturizing skin care wipe, which can be sold as a dry, water-activated wipe with a moisturizing formula containing, in addition to PEO, known emollients and moisturizers. For instance, such a wipe may comprise vinyl pyrrolidone copolymers as the support polymer, PEO in an amount of 3 wt. % to 7 wt. %, moisturizing ingredients such as emollient esters, glycerin, and fragrance. The fluid composition may be cast on to nonwoven fabrics, foam sheets or other support substrates, solvent removed, and cut into desired sizes.

Another embodiment of the skin care wipes comprises incorporating the fluid composition into or onto a foam sheet to form the erodible anhydrous film. As an example of such an embodiment, a hydrophilic polyurethane foam (HPU) sheet having recesses on at least one surface of the HPU sheet, may be filled with the fluid composition described herein. The HPU can be made up of a water phase and a polymer phase that is mixed together to make a foam in a mold so as to form the recesses. The fluid composition may contain a mix of 15 wt. % polyvinylpyrrolidone (PVP); 5wt. % polyvinylpyrrolidone/vinyl acetate copolymer (PVP/VA); 14 wt. %. PEO; polyamide copolymer emollient; a therapeutic agent such as 1 wt. % salicylic acid or 2 wt. %. benzoyl peroxide; isopropanol, and 2 wt. % surfactant(s). This fluid composition can be cast into the foam sheet settling into the recesses and dried to remove the isopropanol solvent. Alternatively, the foam sheet can be saturated with the fluid composition and dried such that the erodible anhydrous film resides on a surface of the foam sheet and/or in the interstitial spaces of the foam sheet.

Yet another embodiment of the erodible anhydrous film contemplates hair care wherein a comb or brush comprises the erodible anhydrous film disclosed herein. The erodible anhydrous film is cast or molded into strips or strings for wrapping around the comb or brush "teeth", or form the actual "teeth" itself. A user then runs the comb through wet hair so that water-soluble active agents within the erodible anhydrous film associated or attached to the comb/brush, including the PEO, are released onto the hair surface. Alternatively, the user can activate the erodible anhydrous film with an adequate amount of water and run the comb through dry hair. Fluid compositions can be of the general formula described above wherein PEO is present in an amount of about 14 wt. %, the support polymer is preferably vinyl acetate/butyl maleate/isobornyl acrylate copolymer, and further containing PVP and PVP/VA polymers or other polymers known to one of skill in the art to be useful for treating hair to provide shine, moisture, sun protection, and the like. Preferably, the solvent is ethanol or isopropanol.

In the area of fabric care, another embodiment of the erodible anhydrous film may also be used in the form of or in connection with a dryer sheet or laundry detergent film. The erodible anhydrous film may be used in treating or softening a fabric in a dryer, where the fabric is contacted with the dryer sheet, in a dryer, under conditions of heat and water vapor. For instance, the dryer sheet can comprise of the erodible anhydrous film of the general formula as described above wherein PVP is the support polymer, and additionally about 8 wt. % of mixed solid fabric softener ingredients, fragrance and solid nonionic surfactants, and diatomaceous earth. The fluid composition may be cast onto a nonwoven or other substrate. As a laundry detergent film, the fabric is contacted with the erodible anhydrous film in the presence of water, so as to release fabric cleaning agents into the wash water or treatment materials onto the fabric in a laundry machine during operation. Such a laundry detergent film can include a concentrated water soluble detergent suitable for use in a standard or high efficiency washing machine. For instance, the laundry detergent film can be an erodible anhydrous film of the general formula as described above, but with a minimum amount of the support polymer or a water soluble support polymer can be selected. Preferably, the support polymer is water soluble, and can comprise a PVP polymer. The formula can also contain PEO in an amount of about 15 wt. %, as well as laundry detergent ingredients comprising surfactants, enzymatic cleaners, fabric brighteners, fragrance, and the like.

In some embodiments, the erodible anhydrous film can be used in treating or cleaning hard surfaces such as floors, bathroom or kitchen surfaces and countertops, and the like. Using the erodible anhydrous film in the form of or in connection with a hard surface cleaner, this method entails the step of contacting the hard surface with the erodible anhydrous film under conditions of wetness, or applying a solution comprising the erodible anhydrous film dissolved in water to the hard surface. For instance, the hard surface cleaning film can be made of the general formula of the invention as described above and further including a concentrated amount of one or more water soluble detergents such that a user would dissolve the erodible anhydrous film in an amount of water for cleaning the hard surface such as the floor or counter top. Preferably, the support polymer is water soluble and can comprise a PVP polymer, the PEO can be in an amount of about 2.5%, and other ingredients can be included such as surfactants including cationic, anionic, non-ionic, and/or zwitterionic surfactants, de-greasers, and the like.

### EXAMPLES

### Erodible Anhydrous Films Prepared by Solvent Casting

Fluid compositions were prepared by heating a beaker equipped with a heating element and stirrer and containing 2-propanol to 45 °C adding the desired amount of SYLVACLEAR® A200V and stirring until the SYLVACLEAR® A200V was dissolved. To this clear solution is added, with stirring, DOW CORNING Elastomer 9546 followed by SILFORM® flexible resin. The solution is then cooled to room temperature, the support polymer, ADVANTAGE® PLUS vinyl acetate/butyl maleate/isobornyl acrylate terpolymer, is added with mixing for approximately 15 minutes after which powdered PEO is slowly added until the PEO is fully dispersed in the mixture with little clumping. To this dispersion is added polyethylene glycol-200 with stirring for approximately 10 minutes followed by the addition of CERAPHYL® 375 with stirring for approximately another 10 minutes. Additional isopropanol is added to adjust the viscosity to about 1200 MPa●s (cPs) to about 2000 MPa●s (cPs).

A stainless steel plate having a thickness of 3.175 mm (1/8 inch) is fitted with a sheet of silicone treated release paper on the top side. Using an appropriate doctor blade, the fluid composition is poured in front of the doctor blade across the paper and drawn down the fluid composition. The cast fluid composition is then dried in a convection oven at about 80 °C to about 85 °C until a constant weight is achieved. Using the appropriate doctor blade, dried films having thicknesses of 1 mm, 1.5 mm, and 2 mm are prepared.

Exemplary embodiments of the fluid composition comprising ADVANTAGE® PLUS vinyl acetate/butyl maleate/isobornyl acrylate terpolymer useful for preparing the erodible anhydrous films are shown in Table II.

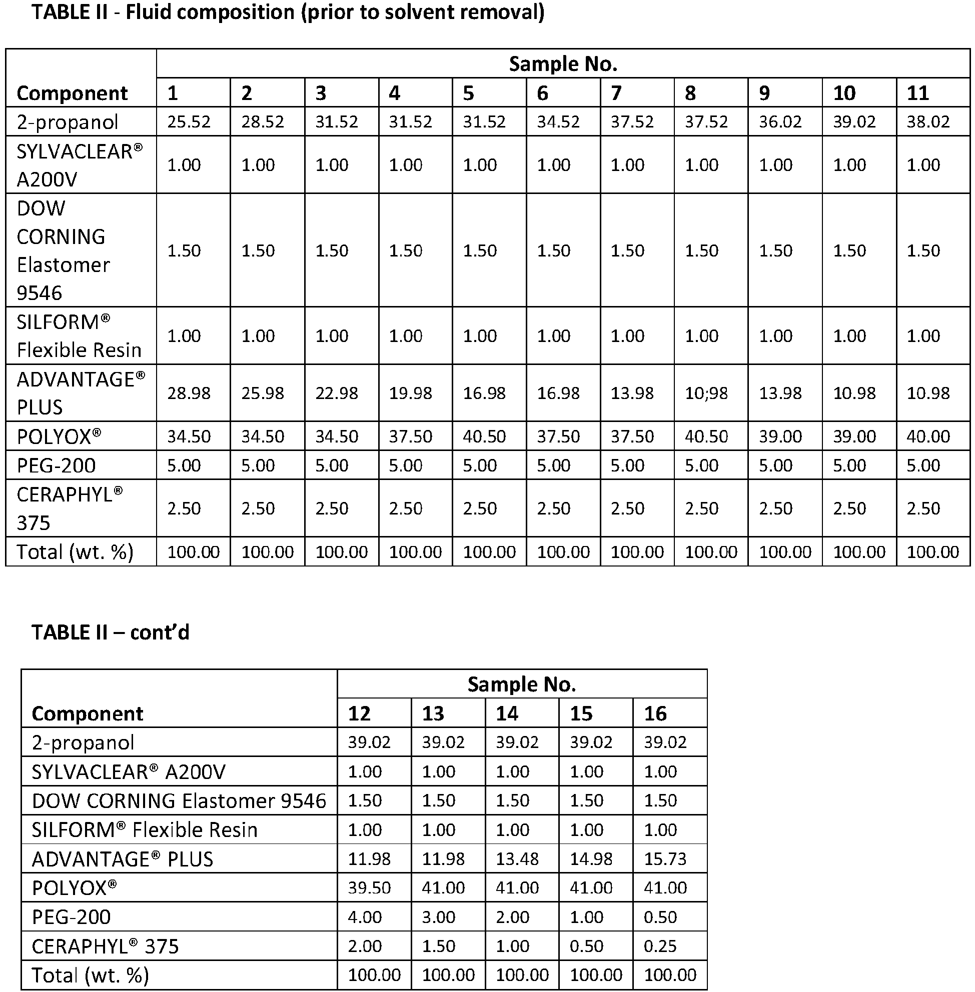

The fluid compositions of Samples 1 to 16 were cast and the solvent removed to form a solid erodible anhydrous film. The components of the resultant erodible anhydrous film are shown in Table III.

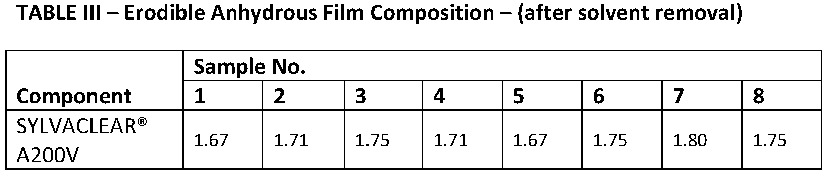

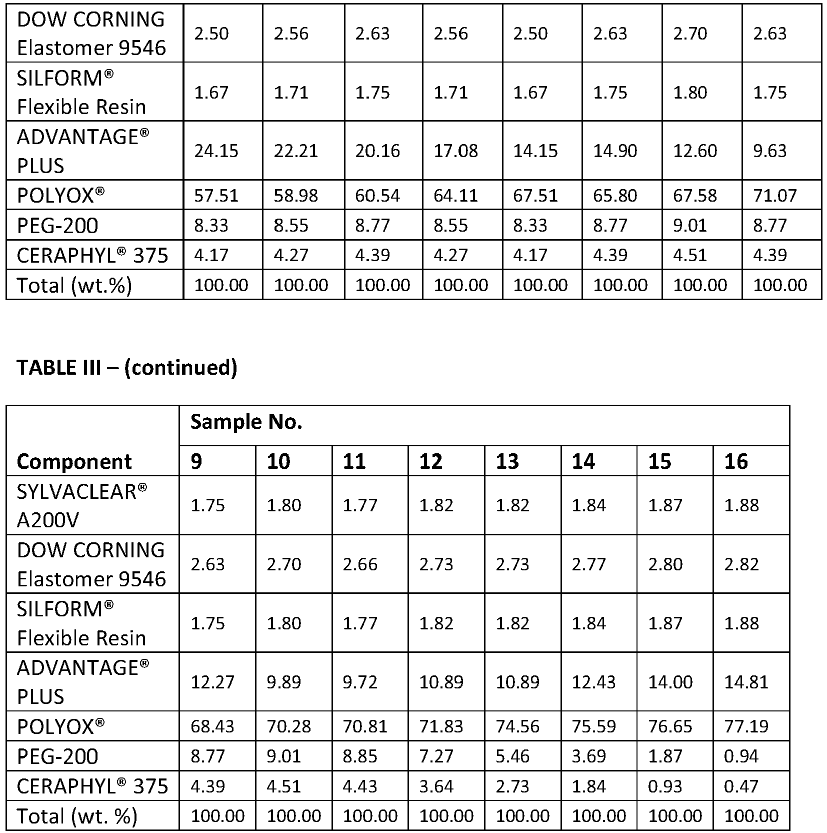

Fluid compositions comprising PEO and a commercial acrylate polymer as shown in Table IV below were prepared varying select components and amounts to adjust the resultant erodible anhydrous film properties.

**TABLE IV - Additional fluid compositions (prior to solvent removal)**

| **Component** | **Sample No.** | | | | |
|---|---|---|---|---|---|
| | **17** | **18** | **19** | **20** | **21** |
| GIOVAREZ® polymer ^{∼}30 wt% in isododecane | 58.00 | 58.00 | 58.00 | 58.00 | 58.00 |
| SYLVACLEAR® C-75V | 1.00 | -- | -- | -- | -- |
| SYLVACLEAR® PE 1800V | -- | -- | -- | 1.00 | -- |
| SYLVACLEAR® A 200V | -- | -- | -- | -- | 1.00 |
| DOW CORNING ST Wax 30 | -- | -- | 1.00 | -- | -- |
| DOW CORNING SW-8005 C30 Resin Wax | -- | 1.00 | -- | -- | -- |
| DOW CORNING Elastomer EL-8051 | 1.50 | 1.50 | 1.50 | 1.50 | 1.50 |
| SILFORM® Resin | 2.50 | 2.50 | 2.50 | 2.50 | 2.50 |
| POLYOX® | 34.50 | 34.50 | 34.50 | 34.50 | 34.50 |
| CERAPHYL® 375 | 2.50 | 2.50 | 2.50 | 2.50 | 2.50 |
| Total (wt.%) | 100.00 | 100.00 | 100.00 | 100.00 | 100.00 |

**TABLE V - Additional Erodible Anhydrous Film Composition (after solvent removal)**

| **Component** | **Sample No.** | | | | |
|---|---|---|---|---|---|
| | **17** | **18** | **19** | **20** | **21** |
| GIOVAREZ® polymer | 40.84 | 40.84 | 40.84 | 40.84 | 40.84 |
| SYLVACLEAR® C-75V | 1.41 | -- | -- | -- | -- |
| SYLVACLEAR® PE 1800V | -- | -- | -- | 1.41 | -- |
| SYLVACLEAR® A 200V | -- | -- | -- | -- | 1.41 |
| DOW CORNING ST Wax 30 | -- | -- | 1.41 | -- | -- |
| DOW CORNING SW-8005 C30 Resin Wax | -- | 1.41 | -- | -- | -- |
| DOW CORNING Elastomer EL-8051 | 2.11 | 2.11 | 2.11 | 2.11 | 2.11 |
| SILFORM® Resin | 3.52 | 3.52 | 3.52 | 3.52 | 3.52 |
| POLYOX® | 48.60 | 48.60 | 48.60 | 48.60 | 48.60 |
| CERAPHYL® 375 | 3.52 | 3.52 | 3.52 | 3.52 | 3.52 |
| Total (wt.%) | 100.00 | 100.00 | 100.00 | 100.00 | 100.00 |

### Example 22 Solvent Cast Erodible Anhydrous Film Comprising PEO and Polyurethane

A solution of 18 grams of LUBRIZOL SP 80A-150 polyurethane polymer and 12 grams of LUBRIZOL SG 80A polyurethane polymer in 190 grams of tetrahydrofuran was prepared as mixture A. To 11 grams of mixture A was added a dispersion comprising 14.25 grams of polyethylene oxide particles, having a particle size of less than 53 µm, in 13.5 grams of tetrahydrofuran(mixture B). The resulting composition was mixed, poured into a frame and allowed to air dry for 40 minutes for the solvent to evaporate. A layer of pressure sensitive adhesive with an associated liner was placed on the film, the film was then removed from the frame and further dried in an oven at 80°C for 24 hours. The PEO/polyurethane film comprised 11 wt. % polyurethane and 89 wt. % PEO. When the procedure was repeated using 22 grams of mixture A and mixture B provided above, the resultant PEO/polyurethane film comprised 22 wt. % polyurethane and 78 wt. % PEO.

### Solvent Cast Erodible Anhydrous Films With Various Support Polymers

Fluid compositions comprising PEO and various support polymer chemistry were prepared according to procedures analogous to that of the preceding Examples 1 to 16 and then converted to erodible anhydrous cast films. The following support polymers, as shown in Table VI were used:
- PVP K-90: polyvinylpyrrolidone
- DERMACRYL® 2.0: acrylates/octyl acrylates
- DERMACRYL® 79: acrylates/octyl acrylates
- GANEX® V-220-F: vinylpyrrolidone/eicosene copolymer
- LUVISET® CAN: vinyl acetate/crotonates/vinyl neodecanoate copolymers
- FLEXAN® II: sodium polystyrene sulfonate
- AQUAFLEX® SF-40: vinylpyrrolidone/vinyl caprolactam/DMAPA acrylates copolymer

**TABLE VI**

| **Component** | **23** | **24** | **25** | **26** | **27** | **28** | **29** |
|---|---|---|---|---|---|---|---|
| 2-propanol | 44.31 | 44.31 | 44.31 | 44.31 | 44.31 | 44.31 | 13.58 |
| SYLVACLEAR® A200V | 1.00 | 1.00 | 1.00 | 1.00 | 1.00 | 1.00 | 1.00 |
| DOW CORNING Elastomer 9546 | 1.50 | 1.50 | 1.50 | 1.50 | 1.50 | 1.50 | 1.50 |
| SILFORM® Flexible Resin | 1.00 | 1.00 | 1.00 | 1.00 | 1.00 | 1.00 | 1.00 |
| PVP K-90 | 5.49 | --- | --- | --- | --- | --- | --- |
| DERMACRYL® 2.0 | --- | 5.49 | --- | --- | --- | --- | --- |
| DERMACRYL® 79 | --- | --- | 5.49 | --- | --- | --- | ---- |
| GANEX® V-220-F | --- | --- | --- | 5.49 | --- | --- | --- |
| LUVISET® CAN | --- | --- | --- | --- | 5.49 | --- | --- |
| FLEXAN® II | --- | --- | --- | --- | --- | 5.49 | --- |
| AQUAFLEX® SF-40 | | | | | | | 36.22 |
| PEO COAGULANT | 39.00 | 39.00 | 39.00 | 39.00 | 39.00 | 39.00 | 39.00 |
| PEG-200 | 5.00 | 5.00 | 5.00 | 5.00 | 5.00 | 5.00 | 5.00 |
| CERAPHYL® 375 | 2.50 | 2.50 | 2.50 | 2.50 | 2.50 | 2.50 | 2.50 |
| Aloe | 0.10 | 0.10 | 0.10 | 0.10 | 0.10 | 0.10 | 0.10 |
| Vitamin E | 0.10 | 0.10 | 0.10 | 0.10 | 0.10 | 0.10 | 0.10 |
| Total (wt. %) | 100.00 | 100.00 | 100.00 | 100.00 | 100.00 | 100.00 | 100.00 |

### Anhydrous Erodible Anhydrous Films Prepared by Injection Molding

Erodible anhydrous films comprising PEO and methacrylic acid-ethyl acrylate copolymer, EUDRAGIT® L100-55 were prepared by injection molding. The formulations and processing temperatures are shown in Table VII.

**Table VII Formulas containing poly(meth)acrylate polymers for injection molding**

| **Ingredient** | **30** | **31** | **32** | **33** | **34** |
|---|---|---|---|---|---|
| EUDRAGIT® L100-55 | 46.7 | 31.2 | 31.2 | 31.2 | 28.5 |
| Triethyl citrate | 13.3 | 8.8 | 8.8 | 8.8 | 1.5 |
| PEO coagulant | 40 | 37 | 40 | 40 | 50.0 |
| Stearic acid | -- | | | 20 | 20.0 |
| Polyox® WSR N750 | | 18 | | | |
| PEG 4600 | | 5 | | | |
| HOSTAPON® SCI-85P | | | | | |
| Glycerol monostearate (TEGIN® M, Evonik) | | | 20 | | |
| Processing | Short part, rubbery texture, processed at 250°F | Rubbery part | Processed well at 240°F | Molded well at 190°F, strong part | Molds well |

Analogous erodible anhydrous films were also prepared in substituting for the EUDRAGIT® L100-55 polymer in one series a 1:1 methacrylic acid-methyl methacrylate copolymer, i.e., EUDRAGIT® L100, and in another series a 2:1 methacrylic acid-methyl methacrylate copolymer, i.e., EUDRAGIT® S100.

EUDRAGIT® formulations containing triethyl citrate plasticizer and stearic acid as processing aid can be molded at temperatures under 200°F. The formulation of Example 30 without stearic acid did not provide a complete fill of part (or perhaps the part shrunk upon release), had a rubbery texture and required higher process temperatures. Use of processing aids such as POLYOX® WSR N750 and PEG 4600 in Example 31 likewise provided a part with a rubbery texture which was difficult to mold. Example 33 comprising stearic acid and glycerol monostearate process aids molded well at the low temperature of 190°F and provided a strong part which processed well.

At a minimum, all of these compositions released PEO when in contact with water, and in simulated shaving conditions. Many of the compositions released PEO and the other shaving aid materials (1) controllably, (2) at a consistent rate, and (3) sustainably even after multiple wet uses.

### Lubricity and Wear

The erodible anhydrous films of Example 1 comprising polyethylene oxide and a vinyl acetate/butyl maleate/isobornyl acrylate terpolymer, prepared by solvent casting, were incorporated as comfort strips on a five-bladed razor cartridge and evaluated for maintenance of perceived lubrication and shaving performance against a razor cartridge comprising a reservoir of gel forming ultrasonically compressed polyethylene oxide held within a non-erodible substrate (i.e., similar to a SCHICK® HYDRO® razor sold by Edgewell Personal Care, LLC) comprising a series of openings that allow release of the PEO.

The evaluation employed a panel of 22 male shavers who had participated in three previous lubrication validation studies. Each panelist was given two razors, identified only by a three digit code number and instructed to shave one half of his face with the first razor and the other half of his face with the second razor, each day for 40 days. One razor in each pair contained a cartridge with a comfort strip comprising the erodible anhydrous film according to Example 1 of the invention, and the second razor contained the cartridge with the comparative comfort technology, i.e., PEO composition in a reservoir. Each panelist arrived at the test facility each day unshaven, shaved at the facility, and then completed a questionnaire rating the razors against each other for glide, cushion, stickiness, oiliness while shaving, stringiness, pulling or tugging, scraping, safety, overall performance, residue while shaving, closeness, irritation, smoothness, oiliness after rinsing, and amount of residue after rinsing.

There was little statistically significant difference noted over time for many of the qualities tested. However, as the test progressed, i.e., after several shaves, the razor comprising the erodible anhydrous film of the invention as a comfort strip was perceived to exhibit less scraping, less irritation, less pulling or tugging, and a greater amount of cushion and glide than the razor comprising the reservoir of gel forming comfort composition. These perceptions were generally more prominent in the half shaves from day 1 through day 20, but were less prominent after day 20 to day 40 indicating that the erodible anhydrous comfort strip of the invention maintained its activity better than the comparative reservoir of gel forming composition though approximately 20 half shaves. That is, the comparative comfort technology lost activity through the first number of shaves to a greater degree than the inventive erodible anhydrous comfort strip, and the loss of properties of the inventive erodible anhydrous strip only started to approach a similarly noticeable degree of property loss after 20 half shaves.

Example 10 was also evaluated for lubrication and shaving performance when incorporated into a four-bladed razor cartridge against a SCHICK® QUATTRO® razor (sold by Edgewell Personal Care, LLC), that includes a traditional extruded matrix comfort strip.

The evaluation employed a panel of 120 male shavers who completed five (5) full shaves with each product in a balanced sequential monadic design. After each shave, the panelists completed a questionnaire on the performance of each product. After five full shaves, the razor with the erodible anhydrous film of Example 10 had highly significant greater mean scores (99% Level of Confidence) for such attributes as shaving comfort, lubrication quality, razor glide, less pull and tug, and lower irritation.

As can be seen from the above examples, erodible anhydrous films of the invention are readily prepared and provide consistent release of polyethylene oxide and ancillary components over an extended period of time. The use of the erodible anhydrous films as a means for transferring desired materials to a surface are also shown to have advantages, especially in terms of longer useful life, over other similar technologies.

## Claims

1. An erodible anhydrous film comprising, based on a total weight of said erodible anhydrous film,
40 wt. % to 80 wt. % of polyethylene oxide, wherein at least a portion of the polyethylene oxide is one or more polyethylene oxide polymers having a molecular weight of 2,000,000 to 10,000,000;
5 wt. % to 40 wt. % of one or more support polymers; and
one or more agents selected from therapeutic agents, cleaning agents, or processing aids,
**characterized in that** the one or more support polymers comprises one or more (meth)acrylate/(meth)acrylic acid copolymer, monoalkyl ester of polymethyl vinyl ether/maleic anhydride copolymer, vinyl pyrrolidone/ dimethylaminoethylmethacrylate copolymer, behenyl methacrylate/*t*-butyl methacrylate copolymer, vinyl acetate/butyl maleate/isobornyl acrylate terpolymer, alone or in combination thereof.

2. The erodible anhydrous film of claim 1, wherein the therapeutic agents comprise one or more of aloe, vitamins, humectants, emollients, moisturizers, clotting agents, anti-chafing agents, fragrances, depilatory agents, essential oils, antioxidants, alpha-hydroxy acids, alpha-keto acids, anti-bacterials, anti-fungals, anti-microbials, anti-virals, analgesics, anti-allergenics, antihistamines, anti-inflammatory agents, anti-irritants, anti-neoplastics, immune boosting agents, immune suppressing agents, anti-acne agents, anti-aging agents, anesthetics, antiseptics, insect repellents, skin cooling compounds, skin warming compounds, skin protectants, skin penetration enhancers, exfoliants, lubricants, or combinations thereof.

3. The erodible anhydrous film of claim 1 or 2, wherein the cleaning agents comprise de-greasers, surfactants, enzymes, fabric conditioners, anti-microbials, anti-bacterials, anti-fungals, or combinations thereof, and
the processing aids comprise plasticizers, which plasticizers comprise polyethylene glycol, propylene glycol, butylene glycol, pentalene glycol, glycerin or combinations thereof, or wherein the processing aids comprise flexibilizers, which flexibilizers comprise silicone resins, waxes, elastomers, polyamides, or combinations thereof, or
wherein the processing aids comprise fillers, which fillers comprise boron nitride, graphite, talc, clay, silicates, diatomaceous earth, or combinations thereof.

4. The erodible anhydrous film of claim 1, comprising a combination of PEO with polyvinylpyrrolidone and/or glycerol and/or polyalkylene glycol.

5. The erodible anhydrous film of claim 1, wherein the one or more support polymer is present in an amount of 6 wt. % to 35 wt. %.

6. The erodible anhydrous film of any one of claims 1 through 5 obtained by a process comprising extruding, injection molding, and or compression molding an anhydrous composition comprising said polyethylene oxide, said one or more support polymers, and said one or more active agents selected from therapeutic agents, cleaning agents and /or processing aids.

7. A process for making the erodible anhydrous film of any one of claims 1 through 6 said process comprising
providing an anhydrous fluid composition comprising,
polyethylene oxide, wherein at least a portion of the polyethylene oxide is one or more polyethylene oxide polymers having a molecular weight of 2,000,000 to 10,000,000 daltons;
one or more support polymer, wherein the one or more support polymers comprises one or more (meth)acrylate/(meth)acrylic acid copolymer, monoalkyl ester of polymethyl vinyl ether/maleic anhydride copolymer, vinyl pyrrolidone/ dimethylaminoethylmethacrylate copolymer, behenyl methacrylate/*t*-butyl methacrylate copolymer, vinyl acetate/butyl maleate/isobornyl acrylate terpolymer, alone or in combination thereof;
one or more active agents selected from therapeutic agents, cleaning agents, or processing aids; and
one or more anhydrous solvent, wherein the one or more anhydrous solvent is present in an amount of 15 wt. % to 55 wt. %, based on a total weight of the anhydrous fluid composition;
casting the erodible anhydrous film by pouring the anhydrous fluid composition onto a substrate or into a mold or into a cavity in an end use device or a component of an end use device; and
drying the anhydrous fluid thus cast.

8. The process of claim 7, wherein the one or more solvent of the anhydrous fluid composition comprises one or more C₂ to C₁₂ alcohols, one or more C₅ to C₂₀ alkanes, one or more C₅ to C₂₀ alkenes, or one or more ethers.

9. The process of claim 7 further comprising water in an amount of less than 5 wt. %, based on a total weight of said anhydrous fluid composition.

10. A multi-layer film comprising one or more layers of the erodible anhydrous film of any one of claims 1 through 6.

11. A skin care wipe, a wound care device, hair care device, fabric care device or an erodible shaving aid material comprising the erodible anhydrous film of any one of claims 1 through 6.

12. A hair removal device comprising the erodible shaving aid material of claim 11.

## Patentansprüche

1. Erodierbarer anhydrischer Film, umfassend, bezogen auf ein Gesamtgewicht des erodierbaren anhydrischen Films,
40 Gew.-% bis 80 Gew.-% Polyethylenoxid, wobei mindestens ein Teil des Polyethylenoxids ein oder mehrere Polyethylenoxidpolymere mit einem Molekulargewicht von 2 000 000 bis 10 000 000 ist;
5 Gew.-% bis 40 Gew.-% eines oder mehrerer Trägerpolymere; und
ein oder mehrere Mittel, ausgewählt aus therapeutischen Mitteln, Reinigungsmitteln oder Verarbeitungshilfsmitteln,
**dadurch gekennzeichnet, dass** das eine oder die mehreren Trägerpolymere ein oder mehrere (Meth)acrylat/(Meth)acrylsäure-Copolymer, Monoalkylester von Polymethylvinylether/Maleinsäureanhydrid-Copolymer, Vinylpyrrolidon/Dimethylaminoethylmethacrylat-Copolymer, Behenylmethacrylat/*t-*Butylmethacrylat-Copolymer, Vinylacetat/Butylmaleat/Isobornylacrylat-Terpolymer einzeln oder in Kombination davon umfassen.

2. Erodierbarer anhydrischer Film nach Anspruch 1, wobei die therapeutischen Mittel ein oder mehrere von Aloe, Vitaminen, Feuchthaltemitteln, Weichmachern, Feuchtigkeitsspendern, Gerinnungsmitteln, Anti-Scheuermitteln, Duftstoffen, Haarentfernungsmitteln, ätherischen Ölen, Antioxidantien, alpha-Hydroxysäuren, alpha-Ketosäuren, antibakteriellen Mitteln, Fungiziden, antimikrobiellen Mitteln, anti-viralen Mitteln, Analgetika, Antiallergika, Antihistaminika, entzündungshemmenden Mitteln, Reiz-mildernden Mitteln, Anti-Neoplastika, Immunstärkungsmittel, Immunsuppressiva, Anti-Akne-Mitteln, Anti-Aging-Mitteln, Anästhetika, Antiseptika, Insektenschutzmitteln, Haut-kühlenden Verbindungen, Haut-wärmenden Verbindungen, Hautschutzmitteln, Hautpenetrationsverstärkern, Peelingmitteln, Gleitmitteln oder Kombinationen davon umfassen.

3. Erodierbarer anhydrischer Film nach Anspruch 1 oder 2, wobei die Reinigungsmittel Entfettungsmittel, Tenside, Enzyme, Weichspüler, antimikrobielle Mittel, antibakterielle Mittel, Fungizide oder Kombinationen davon umfassen, und
die Verarbeitungshilfsmittel Weichmacher umfassen, wobei die Weichmacher Polyethylenglykol, Propylenglykol, Butylenglykol, Pentalenglykol, Glycerin oder Kombinationen davon umfassen, oder
wobei die Verarbeitungshilfsmittel Elastifikatoren umfassen, wobei die Elastifikatoren Silikonharze, Wachse, Elastomere, Polyamide oder Kombinationen davon umfassen, oder
wobei die Verarbeitungshilfsmittel Füllstoffe umfassen, wobei die Füllstoffe Bornitrid, Graphit, Talkum, Ton, Silikate, Kieselgur oder Kombinationen davon umfassen.

4. Erodierbarer anhydrischer Film nach Anspruch 1, umfassend eine Kombination von PEO mit Polyvinylpyrrolidon und/oder Glycerin und/oder Polyalkylenglykol.

5. Erodierbarer anhydrischer Film nach Anspruch 1, wobei das eine oder die mehreren Trägerpolymere in einer Menge von 6 Gew.-% bis 35 Gew.-% vorhanden sind.

6. Erodierbarer anhydrischer Film nach einem der Ansprüche 1 bis 5, erhalten durch ein Verfahren, das Extrudieren, Spritzgießen und/oder Formpressen einer anhydrischen Zusammensetzung umfasst, die das Polyethylenoxid, das eine oder die mehreren Trägerpolymere und den einen oder die mehreren Wirkstoffe, ausgewählt aus therapeutischen Mitteln, Reinigungsmitteln und/oder Verarbeitungshilfsmitteln, umfasst.

7. Verfahren zur Herstellung des erodierbaren anhydrischen Films nach einem der Ansprüche 1 bis 6, wobei das Verfahren umfasst
Bereitstellen einer anhydrischen Fluidzusammensetzung, umfassend Polyethylenoxid, wobei mindestens ein Teil des Polyethylenoxids ein oder mehrere Polyethylenoxidpolymere mit einem Molekulargewicht von 2 000 000 bis 10 000 000 Dalton ist;
ein oder mehrere Trägerpolymere, wobei das eine oder die mehreren Trägerpolymere ein oder mehrere (Meth)acrylat/(Meth)acrylsäure-Copolymer, Monoalkylester von Polymethylvinylether/Maleinsäureanhydrid-Copolymer, Vinylpyrrolidon/Dimethylaminoethylmethacrylat-Copolymer, Behenylmethacrylat/*t-*Butylmethacrylat-Copolymer, Vinylacetat/Butylmaleat/Isobomylacrylat-Terpolymer einzeln oder in Kombination davon umfassen;
einen oder mehrere Wirkstoffe, ausgewählt aus therapeutischen Mitteln, Reinigungsmitteln oder Verarbeitungshilfsmitteln; und
ein oder mehrere anhydrische Lösungsmittel, wobei das eine oder die mehreren anhydrischen Lösungsmittel in einer Menge von 15 Gew.-% bis 55 Gew.-%, bezogen auf ein Gesamtgewicht der anhydrischen Fluidzusammensetzung, vorhanden sind;
Gießen des erodierbaren anhydrischen Films durch Schütten der anhydrischen Fluidzusammensetzung auf ein Substrat oder in eine Form oder in einen Hohlraum in einer Endverwendungsvorrichtung oder einer Komponente einer Endverwendungsvorrichtung; und
Trocknen der so gegossenen anhydrischen Flüssigkeit.

8. Verfahren nach Anspruch 7, wobei das eine oder die mehreren Lösungsmittel der anhydrischen Fluidzusammensetzung ein oder mehrere C₂- bis C₁₂-Alkohole, ein oder mehrere C₅ bis C₂₀-Alkane, ein oder mehrere C₅ bis C₂₀-Alkene oder einen oder mehrere Ether umfassen.

9. Verfahren nach Anspruch 7, weiter umfassend Wasser in einer Menge von weniger als 5 Gew.-%, bezogen auf ein Gesamtgewicht der anhydrischen Fluidzusammensetzung.

10. Mehrschichtiger Film, umfassend eine oder mehrere Schichten des erodierbaren anhydrischen Films nach einem der Ansprüche 1 bis 6.

11. Hautpflegetuch, eine Wundpflegevorrichtung, eine Haarpflegevorrichtung, eine Gewebepflegevorrichtung oder ein erodierbares Rasierhilfsmaterial, umfassend den erodierbaren anhydrischen Film nach einem der Ansprüche 1 bis 6.

12. Haarentfernungsvorrichtung, umfassend das erodierbare Rasierhilfsmaterial nach Anspruch 11.

## Revendications

1. Film anhydre érodable comprenant, sur la base du poids total dudit film anhydre érodable,
40 % en poids à 80 % en poids d'oxyde de polyéthylène, au moins une portion de l'oxyde d'éthylène étant un ou plusieurs polymères d'oxyde de polyéthylène présentant un poids moléculaire de 2 000 000 à 10 000 000 ;
5 % en poids à 40 % en poids d'un ou de plusieurs polymères de support ; et
un ou plusieurs agents sélectionnés parmi des agents thérapeutiques, des agents nettoyants ou des auxiliaires de traitement,
**caractérisé en ce que** le ou les polymères de support comprennent un ou plusieurs copolymères de (méth)acrylate / acide (méth)acrylique, copolymères de monoalkylester de polyméthylvinyléther / anhydride maléique, copolymères de vinylpyrrolidone / méthacrylate de diméthylaminoéthyle, copolymères de méthacrylate de béhényle / méthacrylate de *t*-butyle, terpolymères d'acétate de vinyle / maléate de butyle / acrylate d'isobornyle, seuls ou en combinaison.

2. Film anhydre érodable de la revendication 1, dans lequel les agents thérapeutiques comprennent un ou plusieurs agents parmi de l'aloès, des vitamines, des humectants, des émollients, des agents hydratants, des agents coagulants, des agents anti-irritation, des parfums, des agents dépilatoires, des huiles essentielles, des antioxydants, des alpha-hydroxyacides, des alpha-cétoacides, des antibactériens, des antifongiques, des antimicrobiens, des antiviraux, des analgésiques, des anti-allergènes, des antihistaminiques, des agents anti-inflammatoires, des anti-irritants, des antinéoplasiques, des agents qui renforcent l'immunité, des agents immunosuppresseurs, des antiacnéiques, des agents anti-âge, des anesthésiants, des antiseptiques, des insectifuges, des composés qui rafraîchissent la peau, des composés qui réchauffent la peau, des agents dermoprotecteurs, des renforçateurs de pénétration dermique, des exfoliants, des lubrifiants ou des combinaisons de ceux-ci.

3. Film anhydre érodable de la revendication 1 ou 2, dans lequel les agents nettoyants comprennent des dégraissants, des tensioactifs, des enzymes, des agents de traitement des tissus, des antimicrobiens, des antibactériens, des antifongiques ou des combinaisons de ceux-ci, et
les auxiliaires de traitement comprennent des plastifiants, lesdits plastifiants comprenant du polyéthylèneglycol, du propylèneglycol, du butylèneglycol, du pentalèneglycol, de la glycérine ou des combinaisons de ceux-ci, ou
dans lequel les auxiliaires de traitement comprennent des agents assouplissants, lesdits agents assouplissants comprenant des résines de silicone, des cires, des élastomères, des polyamides ou des combinaisons de ceux-ci, ou
dans lequel les auxiliaires de traitement comprennent des charges, lesdites charges comprenant du nitrure de bore, du graphite, du talc, de l'argile, des silicates, de la terre à diatomées, ou des combinaisons de ceux-ci.

4. Film anhydre érodable de la revendication 1, comprenant une combinaison de PEO avec de la polyvinylpyrrolidone et/ou du glycérol et/ou du polyalkylèneglycol.

5. Film anhydre érodable de la revendication 1, dans lequel le ou les polymères de support sont présents dans une quantité de 6 % en poids à 35 % en poids.

6. Film anhydre érodable de l'une quelconque des revendications 1 à 5, obtenu par un procédé comprenant l'extrusion, le moulage par injection et/ou le moulage par compression d'une composition anhydre comprenant ledit oxyde de polyéthylène, ledit ou lesdits polymères de support, et ledit ou lesdits agents actifs sélectionnés parmi des agents thérapeutiques, des agents nettoyants et/ou des auxiliaires de traitement.

7. Procédé pour fabriquer le film anhydre érodable selon l'une quelconque des revendications 1 à 6, ledit procédé comprenant les étapes consistant à
fournir une composition fluide anhydre comprenant
de l'oxyde de polyéthylène, où au moins une partie de l'oxyde de polyéthylène est un ou plusieurs polymères d'oxyde de polyéthylène ayant un poids moléculaire de 2 000 000 à 10 000 000 daltons ;
un ou plusieurs polymères de support, où le ou les polymères de support comprennent un ou plusieurs copolymères de (méth)acrylate / acide (méth)acrylique, copolymères de monoalkylester de polyméthylvinyléther / anhydride maléique, copolymères de vinylpyrrolidone / méthacrylate de diméthylaminoéthyle, copolymères de méthacrylate de béhényle / méthacrylate de *t*-butyle, terpolymères d'acétate de vinyle / maléate de butyle / acrylate d'isobornyle, seuls ou en combinaison ;
un ou plusieurs agents actifs sélectionnés parmi des agents thérapeutiques, des agents nettoyants ou des auxiliaires de traitement ; et
un ou plusieurs solvants anhydres, où le ou les solvants anhydres sont présents dans une quantité de 15 % en poids à 55 % en poids, sur la base du poids total de la composition fluide anhydre,
mouler ledit film anhydre érodable en versant la composition fluide anhydre sur un substrat ou dans un moule ou dans une cavité d'un dispositif d'utilisation finale ou dans un composant d'un dispositif d'utilisation finale ; et
sécher le fluide anhydre ainsi coulé.

8. Procédé de la revendication 7, dans lequel le ou les solvants de la composition fluide anhydre comprennent un ou plusieurs alcools en C₂ à C₁₂, un ou plusieurs alcanes en C₅ à C₂₀, un ou plusieurs alcènes en C₅ à C₂₀ ou un ou plusieurs éthers.

9. Procédé de la revendication 7, comprenant en outre de l'eau dans une quantité inférieure à 5 % en poids, sur la base du poids total de ladite composition fluide anhydre.

10. Film multicouches comprenant une ou plusieurs couches du film anhydre érodable de l'une quelconque des revendications 1 à 6.

11. Lingette de soin de la peau, dispositif de soin des plaies, dispositif de soins capillaires, dispositif de soin des tissus ou matériau érodable auxiliaire du rasage comprenant le film anhydre érodable selon l'une quelconque des revendications 1 à 6.

12. Dispositif d'épilation comprenant le matériau érodable auxiliaire du rasage de la revendication 11.
